# FASCICULE DE BREVET EUROPEEN

(11) **EP 2 066 700 B1**
(45) Date de publication et mention de la délivrance du brevet: **11.11.2015**
(21) Numéro de dépôt: 07825188.1
(22) Date de dépôt: 26.09.2007
(51) Int. Cl.: C08B 37/02, A61K 47/36, A61K 31/721

(54) **DEXTRAN FONCTIONNALISE PAR DES AMINO-ACIDES HYDROPHOBES**
MIT HYDROPHOBEN AMINOSÄUREN SUBSTITUIERTES DEXTRAN
DEXTRAN FUNCTIONALIZED WITH HYDROPHOBIC AMINO ACIDS

(30) Priorité: 26.09.2006 WO PCT/IB2006/002666; 29.03.2007 FR 0702316
(43) Date de publication de la demande: 10.06.2009
(73) Titulaire: Adocia, 69003 Lyon (FR)
(72) Inventeur: SOULA, Rémi, 69001 Lyon (FR); SOULA, Gérard, 69330 Meyzieu (FR); SOULA Olivier, 69330 Meyzieu (FR)
(74) Mandataire: Tripoz, Inès
(86) Numéro de dépôt international: PCT/IB2007/002807
(87) Numéro de publication internationale: WO 2008/038111

(56) Documents cités:
- DE-A1- 4 136 324
- US-A- 2 808 405
- US-A- 5 977 076
- US-A1- 2004 131 583
- US-B1- 6 617 456

## Description

La présente invention concerne de nouveaux polymères biocompatibles à base de dextran.

Ces polymères peuvent être utiles notamment pour l'administration de principe(s) actif(s) (PA) aux hommes ou aux animaux dans un but thérapeutique et/ou prophylactique.

La présente invention concerne de nouveaux dérivés du dextran amphiphiles fonctionnalisés par au moins un alpha amino-acide hydrophobe. Ces nouveaux dérivés du dextran ont une bonne biocompatibilité et leur hydrophobicité est facilement modulable sans altérer la biocompatibilité.

Parmi les dextrans amphiphiles, les carboxymethyldextrans de Biodex décrits dans le brevet US6,646,120 sont modifiés par la benzylamine. Ce groupement hydrophobe ne fait pas partie de la famille des alpha aminoacides.

Dellacherie et al. ont également décrits des dextrans amphiphiles (Durand, A. et al., Biomacromolecules 2006, 7, 958-964.)(Durand, Alain et al., Colloid Polym.Sci. 2006, 284, 536-545.) obtenus par réaction des fonctions hydroxyles du dextran sur des epoxydes (phenylglycidyl ether, 1,2-epoxyoctane ou 1,2-epoxydodecane). Les polymères amphiphiles décrits ne sont donc pas fonctionnalisés par des dérivés d'amino-acide.

Bauer et al. décrivent des dextrans fonctionnalisés par des acides gras, C10 à C14, dans le brevet US5,750,678. Les polymères obtenus sont amphiphiles mais ne sont pas modifiés par des acides aminés hydrophobes.

Une revue récente des polymères fonctionnels à base de dextrans (Heinze, Thomas et al., Adv Polym Sci 2006, 205, 199-291.) ne fait pas état de dextran fonctionnalisé par un amino-acide hydrophobe.

L'invention concerne donc un dextran fonctionnalisé caractérisé en ce qu'il répond à la formule générale suivante : 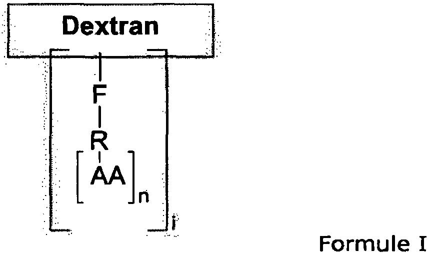
- R étant une chaîne comprenant entre 1 et 18 carbones, éventuellement branchée et/ou insaturée comprenant un ou plusieurs hétéroatomes, et ayant au moins une fonction acide
- F étant soit un ester, un thioester, un amide, un carbonate, un carbamate, un éther, un thioéther ou une amine,
- AA étant un reste amino-acide hydrophobe, L ou D, produit du couplage entre l'amine de l'amino-acide et un acide porté par le groupement R, ledit reste amino-acide étant choisi parmi les dérivés du tryptophane, ou parmi la phénylalanine, la leucine, l'isoleucine et la valine et leurs dérivés alcool, amide ou décarboxylés.

i représente la fraction molaire de substituant F-R-[AA]n par unité glycosidique et est comprise entre 0,1 et 2,

n représente la fraction molaire des R substitués par AA et est comprise entre 0,05 et 1.

Lorsque R n'est pas substitué par AA, alors le ou les acides du groupement R sont des carboxylates de cation, ledit dextran étant amphiphile à pH neutre.

On entend par un reste amino-acide hydrophobe le produit de couplage entre l'amine de l'amino-acide et un acide porté par le groupement R, ledit dextran étant amphiphile à pH neutre.

Selon l'invention le dextran fonctionnalisé répond à la formule générale suivante: 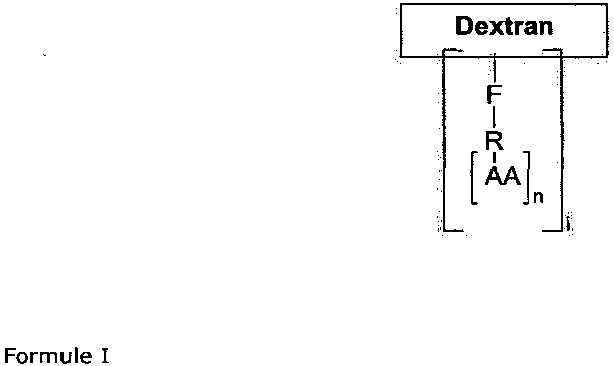
- R étant une chaîne comprenant entre 1 et 18 carbones, éventuellement branchée et/ou insaturée comprenant un ou plusieurs hétéroatomes, tels que O, N ou/et S, et ayant au moins une fonction acide
- F étant soit un ester, un thioester, un amide, un carbonate, un carbamate, un éther, un thioéther ou une amine,
- AA étant un reste amino-acide hydrophobe, L ou D, produit du couplage entre l'amine de l'amino-acide et un acide porté par le groupement R, le reste amino-acide étant choisi parmi les dérivés du tryptophane, ou parmi la phénylalanine, la leucine, l'isoleucine, et la valine, et leurs dérivés alcool, amide ou décarboxylés

i représente la fraction molaire de substituant F-R-[AA]n par unité glycosidique et est comprise entre 0,1 et 2,

n représente la fraction molaire des R substitués par AA et est comprise entre 0,05 et 1,

Lorsque R n'est pas substitué par AA, alors le ou les acides du groupement R sont des carboxylates de cation, alcalin de préférence comme Na, K,
ledit dextran étant amphiphile à pH neutre.

Dans un mode de réalisation, F est soit un ester, un carbonate, un carbamate ou un éther.

Dans un mode de réalisation, le polysaccharide selon l'invention est un carboxymethyl dextran (DMC) de formule IV. 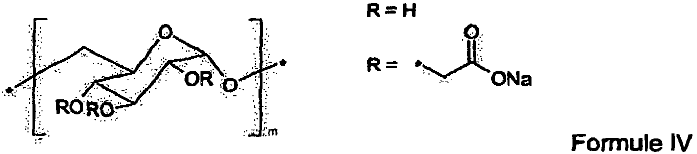 ou l'acide correspondant

Dans un mode de réalisation, le polysaccharide selon l'invention est un ester monosuccinique de dextran ou succinicaciddextran (DSA) de formule V : 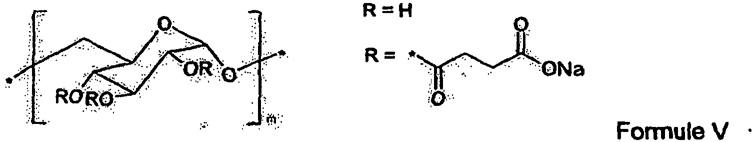 ou l'acide correspondant.

Dans un mode de réalisation, le polysaccharide selon l'invention est caractérisé en ce que le groupe R est choisi dans les groupes suivants : 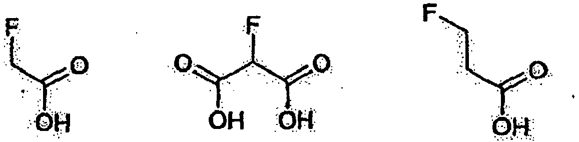 ou leurs sels de cations alcalins.

Dans un mode de réalisation, le dextran selon l'invention est caractérisé en ce que l'amino-acide hydrophobe est choisi parmi les dérivés du tryptophane, tels que le tryptophane, le tryptophanol, le tryptophanamide, le 2-indole éthylamine et leurs sels de cation alcalin.

Dans un mode de réalisation, le dextran selon l'invention est caractérisé en ce que les dérivés du tryptophane sont choisis parmi les esters du tryptophane de formule II

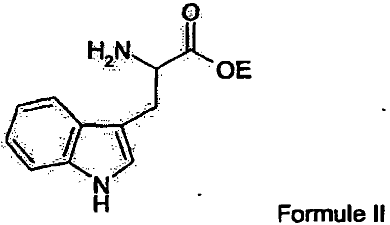

E étant un groupement pouvant être :
- un alkyle linéaire ou ramifié en C1 à C8.
- un alkylaryle ou un arylalkyle linéaire ou ramifié en C6 à C20.

Dans un mode de réalisation le dextran selon l'invention est un carboxyméthyldextran modifié par l'ester éthylique du tryptophane de formule VI :

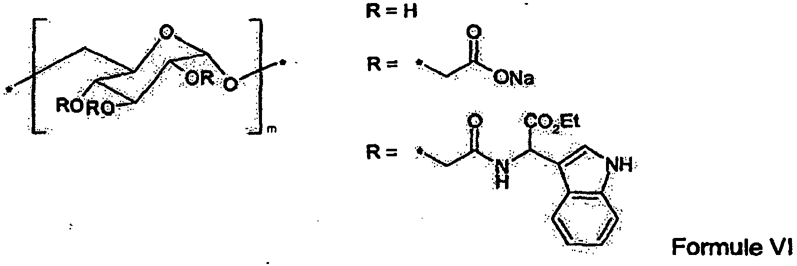

Dans un mode de réalisation le dextran selon l'invention est un ester monosuccinique de dextran ou succinicaciddextran (DSA) modifié par l'ester éthylique du tryptophane de formule VII :

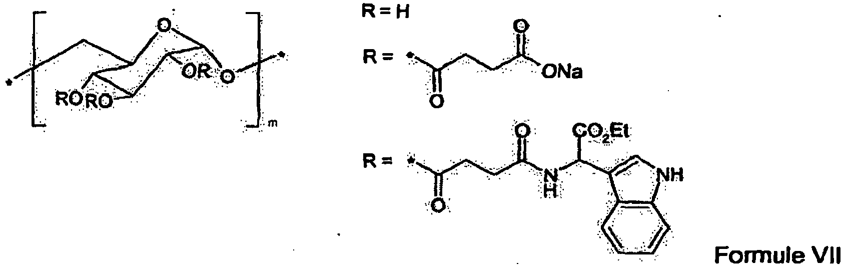

Dans un mode de réalisation, le dextran selon l'invention est caractérisé en ce que l'amino-acide hydrophobe est choisi parmi la phenylalanine, la leucine, l'isoleucine et la valine et leurs dérivés alcool, amide ou décarboxylés.

Dans un mode de réalisation, le dextran selon l'invention est caractérisé en ce que les dérivés de la phenylalanine, de la leucine, de l'isoleucine et de la valine sont choisis parmi les esters de ces acides aminés de formules III.

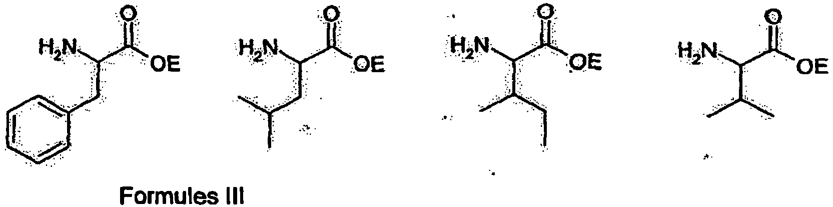

E étant défini comme précédemment.

Le dextran peut avoir un degré de polymérisation m compris entre 10 et 10000.

Dans un mode de réalisation, il a un degré de polymérisation m compris entre 10 et 1000.

Dans un autre mode de réalisation, il a un degré de polymérisation m compris entre 10 et 500.

Les dextrans selon l'invention sont obtenus par greffage d'un ester de l'acide aminé visé sur le dextran modifié par un groupement R.

Dans un mode de réalisation un ester de formule II

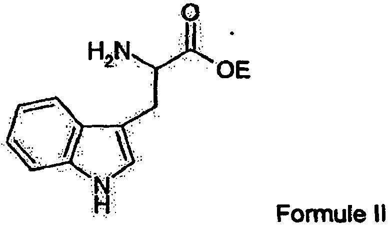

E étant un groupement pouvant être :
- un alkyle linéaire ou ramifié en C1 à C8.
- un alkylaryle ou un arylalkyle linéaire ou ramifié en C6 à C20. est greffé sur un dextran (DMC) de formule IV.

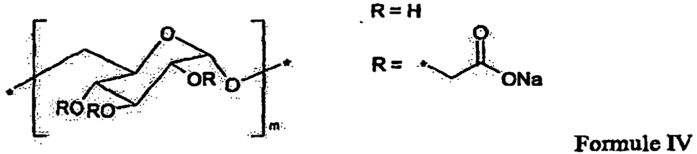

Dans un autre mode de réalisation un ester de formule II telle que définie ci-dessus est greffé sur un dextran (DSA) de formule V.

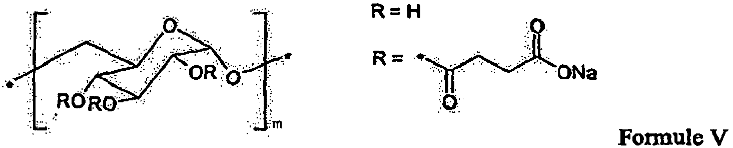

L'invention concerne également une composition pharmaceutique comprenant l'un des dextrans selon l'invention tel que décrit précédemment et au moins un principe actif.

On entend par principe actif un produit sous forme d'entité chimique unique ou sous forme d'une combinaison ayant une activité physiologique. Ledit principe actif peut être exogène c'est à dire qu'il est apporté par la composition selon l'invention. Il peut également être endogène, par exemple les facteurs de croissance qui vont être sécrétés dans une plaie pendant la première phase de cicatrisation et qui pourront être retenus sur ladite plaie par la composition selon l'invention.

L'invention concerne également une composition pharmaceutique selon l'invention telle que décrite précédemment caractérisée en ce qu'elle est administrable par voie orale, nasale, vaginale, buccale.

L'invention concerne également une composition pharmaceutique selon l'invention telle que décrite précédemment, caractérisée en ce qu'elle est obtenue par séchage et/ou lyophilisation.

L'invention concerne également une composition pharmaceutique selon l'invention telle que décrite précédemment, caractérisée en ce qu'elle est administrable sous forme de stent, de film ou « coating » de biomatériaux implantables, d'implant.

L'invention concerne également une composition pharmaceutique selon l'invention telle que décrite précédemment caractérisée en ce que le principe actif est choisi dans le groupe constitué par les protéines, les glycoprotéines, les peptides et les molécules thérapeutiques non-peptidiques.

Les compositions pharmaceutiques possibles sont soit sous forme liquide (nanoparticules ou microparticules en suspension dans l'eau ou dans des mélanges de solvants), soit sous forme de poudre, d'implant ou de film.

Dans le cas des libérations locale et systémique, les modes d'administration envisagés sont par voie intraveineuse, sous-cutanée, intradermique, intramusculaire, orale, nasale, vaginale, oculaire, buccale, etc.

L'invention concerne également l'utilisation des dextrans fonctionnalisés selon l'invention pour la préparation de compositions pharmaceutiques telles que décrites précédemment.

### Exemple 1 : synthèse d'un carboxymethyldextran modifié par l'ester éthylique du tryptophane

Les fonctions acides (i = 1.0) d'un carboxymethyldextran de DP moyen 250 (10 g) sont activées en présence de N-MéthylMorpholine (4.7 g) et de chloroformate d'isobutyl (6.4 g) dans le DMF (180 ml). Le chlorhydrate de l'ester éthylique du tryptophane (5.4 g, Bachem) neutralisé par la TEA (2.0 g) dans le DMF (54 ml) est ensuite greffé sur ce polymère activé à 4°C pendant 45 minutes. Après hydrolyse des acides activés restants (94 ml d'eau), le polymère est dilué dans l'eau (720 ml) et le pH est fixé à 7 par ajout de soude. Le polymère est ensuite purifié par ultrafiltration. Le polymère obtenu a la structure suivante.

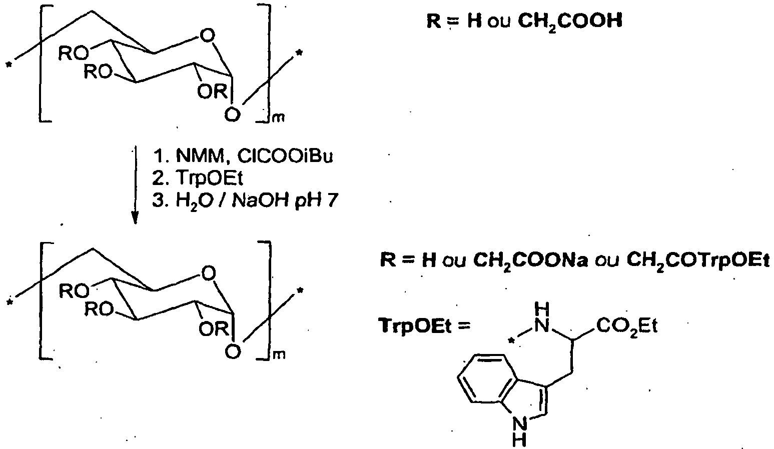

La fraction molaire des acides modifiés par l'ester éthylique du tryptophane est de 0.45 d'après la RMN ¹H dans D₂O/NaOD (n=0.45). La fraction molaire des acides non modifiés et modifiés par unité glycosidique est de 1.0 (i=1.0).

### Exemple 2: synthèse d'un carboxymethyldextran modifié par l'ester méthylique de la leucine

Les fonctions acides (i = 1.0) d'un carboxymethyldextran de DP moyen 250 (10 g) sont activées en présence de N-MéthylMorpholine (4.7 g) et de chloroformate d'isobutyl (6.4 g) dans le DMF (180 ml). Le chlorhydrate de l'ester méthylique de la leucine (3.7 g, Bachem) neutralisé par la TEA (2.0 g) dans le DMF (54 ml) est ensuite greffé sur ce polymère activé à 4°C pendant 45 minutes. Après hydrolyse des acides activés restants (94 ml d'eau), le polymère est dilué dans l'eau (720 ml) et le pH est fixé à 7 par ajout de soude. Le polymère est ensuite purifié par ultrafiltration. Le polymère obtenu a la structure suivante.

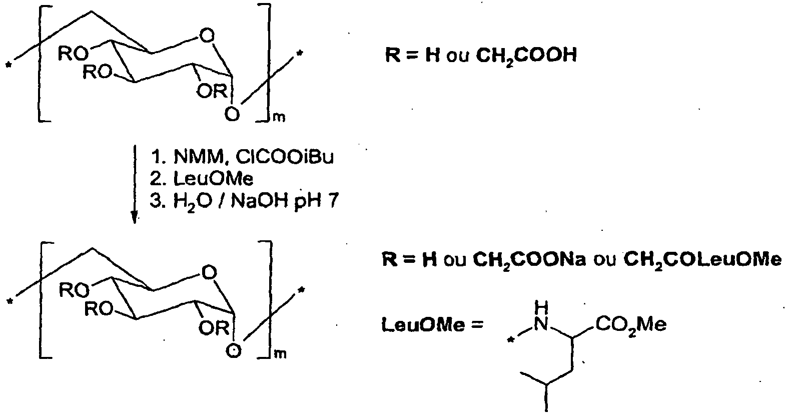

La fraction molaire des acides modifiés par l'ester méthylique de la leucine est de 0.30 d'après la RMN ¹H dans D₂O/NaOD (n=0.30). La fraction molaire des acides non modifiés et modifiés par unité glycosidique est de 1.0 (i=1.0).

### Exemple 3 : synthèse d'un carboxymethyldextran modifié par l'ester éthylique de la phénylalanine

Les fonctions acides (i = 1.0) d'un carboxymethyldextran de DP moyen 250 (10 g) sont activées en présence de N-MéthylMorpholine (4.7 g) et de chloroformate d'isobutyl (6.4 g) dans le DMF (180 ml). Le chlorhydrate de l'ester éthylique de la phénylalanine (4.6 g, Bachem) neutralisé par la TEA (2.0 g) dans le DMF (54 ml) est ensuite greffé sur ce polymère activé à 4°C pendant 45 minutes. Après hydrolyse des acides activés restants (94 ml d'eau), le polymère est dilué dans l'eau (720 ml) et le pH est fixé à 7 par ajout de soude. Le polymère est ensuite purifié par ultrafiltration. Le polymère obtenu a la structure suivante.

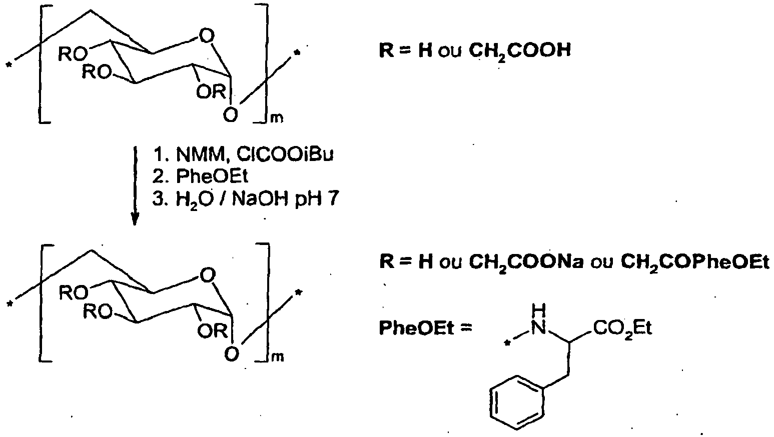

La fraction molaire des acides modifiés par l'ester éthylique de la phénylalanine est de 0.45 d'après la RMN ¹H dans D₂O/NaOD (n=0.45). La fraction molaire des acides non modifiés et modifiés par unité glycosidique est de 1.0 (i=1.0).

### Exemple 4 : synthèse d'un carboxymethyldextran modifié par le sel de sodium du tryptophane

Le polymère obtenu dans l'exemple 1 est mis en solution dans l'eau (30 mg/ml) et le pH est fixé à 12.5 par ajout de soude 1 N. Après une nuit d'agitation à température ambiante, le produit est purifié par ultrafiltration.

La fraction molaire des acides modifiés par le sel de sodium du tryptophane est de 0.45 d'après la RMN ¹H dans D₂O (n=0.45). La fraction molaire des acides non modifiés et modifiés par unité glycosidique est de 1.0 (i=1.0).

### Exemple 5 : synthèse d'un succinicaciddextran modifié par l'ester éthylique du tryptophane

Le dextran de DP moyen 250, D40, (10 g, Amersham Biosciences), est solubilisé dans du DMSO (25 ml) à 40°C. A cette solution sont ajoutés l'anhydride succinique en solution dans du DMF (6.2 g dans 25 ml) et la N-MéthylMorpholine, NMM, diluée dans le DMF (6.2 g dans 25 ml). Après 1 heure de réaction, le milieu réactionnel est dilué dans de l'eau (400 ml) et le polymère est purifié par ultrafiltration. La fraction molaire d'ester succinique formé par unité glycosidique est de 1.0 d'après la RMN ¹H dans D₂O/NaOD (i=1.0).

Du DSA en solution aqueuse (350 g d'une solution à 28 mg/ml) est acidifié sur résine échangeuse d'ions (300 ml de résine humide, Purolite, C100H). La solution obtenue est congelée puis lyophilisée.

Du DSA acidifié (8 g) est solubilisé dans le DMF (115 ml) à température ambiante. A la solution refroidie à 0°C, sont ajoutés le chloroformate d'éthyle (3.3 g) puis la NMM (3.1 g). Le chlorhydrate de l'ester éthylique du tryptophane (3.7 g, Bachem) neutralisé par la TEA (1.4 g) dans le DMF (37 ml) est ensuite ajouté au milieu réactionnel à 4°C et le milieu est agité pendant 45 minutes. Après hydrolyse des acides activés restants, le polymère est dilué dans l'eau (530 ml) et le pH est fixé à 7 par ajout de soude. Le polymère est ensuite purifié par ultrafiltration. Le polymère obtenu a la structure suivante.

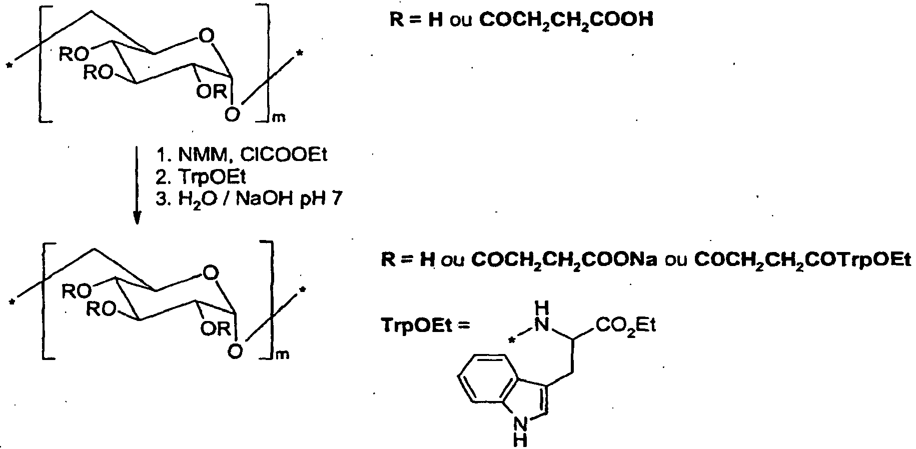

La fraction molaire des acides modifiés par l'ester éthylique du tryptophane est de 0.45 d'après la RMN ¹H dans D₂O/NaOD (n=0.45). La fraction molaire des acides non modifiés et modifiés par unité glycosidique est de 1.0 (i=1.0).

### Exemple 6 : synthèse d'un succinicaciddextran modifié par l'ester éthylique du tryptophane

Le dextran de DP moyen 250, D40, (20 g, Amersham Biosciences) est solubilisé dans du DMSO (50 ml) à 40°C. A cette solution sont ajoutés l'anhydride succinique en solution dans du DMF (24.7 g dans 50 ml) et la N-MéthylMorpholine, NMM, diluée dans le DMF (25.0 g dans 50 ml). Après 3 heures de réaction, le milieu réactionnel est dilué dans de l'eau (800 ml) et le polymère est purifié par ultrafiltration. La fraction molaire d'ester succinique formé par unité glycosidique est de 1.8 d'après la RMN ¹H dans D₂O/NaOD (i=1.8).

Du DSA en solution aqueuse (720 g d'une solution à 29.5 mg/ml) est acidifié sur résine échangeuse d'ions (300 ml de résine humide, Purolite, C100H). La solution obtenue est congelée puis lyophilisée.

Du DSA acidifié (22.3 g) est solubilisé dans le DMF (542 ml) à température ambiante. A la solution refroidie à 0°C, sont ajoutés le chloroformate d'éthyle (13.4 g) puis la NMM (12.5 g). Le chlorhydrate de l'ester éthylique du tryptophane (7.5 g, Bachem) neutralisé par la TEA (2.8 g) dans le DMF (75 ml) est ensuite ajouté au milieu réactionnel à 4°C et le milieu est agité pendant 45 minutes. Après hydrolyse des acides activés restants, le polymère est dilué dans l'eau (530 ml) et le pH est fixé à 7 par ajout de soude. Le polymère est ensuite purifié par ultrafiltration. Le polymère obtenu a la structure suivante.

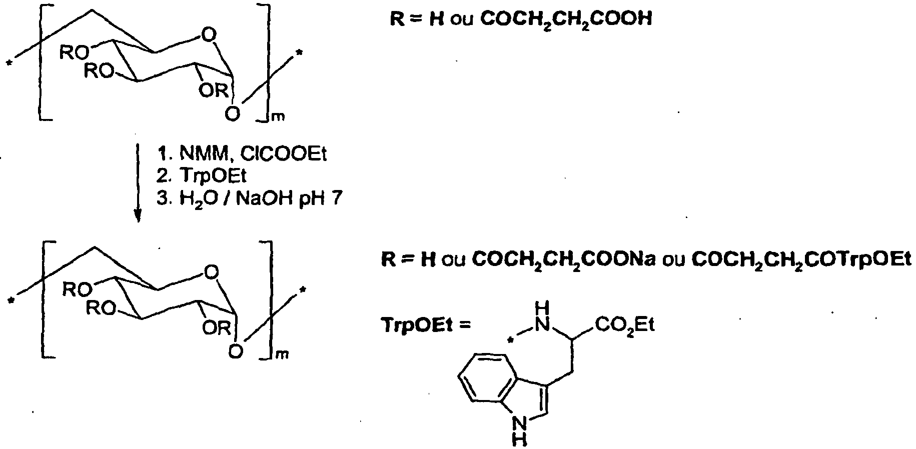

La fraction molaire des acides modifiés par l'ester éthylique du tryptophane est de 0.25 d'après la RMN ¹H dans D₂O/NaOD (n=0.25). La fraction molaire des acides non modifiés et modifiés par unité glycosidique est de 1.8 (i=1.8).

## Revendications

1. Dextran fonctionnalisé **caractérisé en ce qu'**il répond à la formule générale suivante : 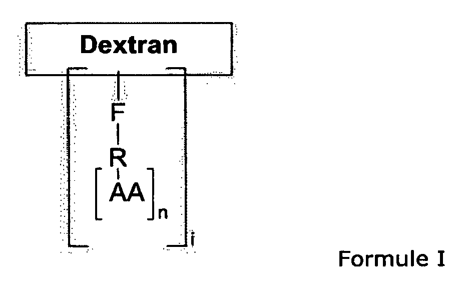
• R étant une chaîne comprenant entre 1 et 18 carbones, éventuellement branchée et/ou insaturée comprenant un ou plusieurs hétéroatomes, et ayant au moins une fonction acide
• F étant soit un ester, un thioester, un amide, un carbonate, un carbamate, un éther, un thioéther ou une amine,
• AA étant un reste amino-acide hydrophobe, L ou D, produit du couplage entre l'amine de l'amino-acide et un acide porté par le groupement R, ledit reste amino-acide étant choisi parmi les dérivés du tryptophane, ou parmi la phenylalanine, la leucine, l'isoleucine et la valine et leurs dérivés alcool, amide ou décarboxylés.
i représente la fraction molaire de substituant F-R-[AA]n par unité glycosidique et est comprise entre 0,1 et 2,
n représente la fraction molaire des R substitués par AA et est comprise entre 0,05 et 1.
Lorsque R n'est pas substitué par AA, alors le ou les acides du groupement R sont des carboxylates de cation,
ledit dextran étant amphiphile à pH neutre.

2. Dextran selon la revendication 1, **caractérisé en ce que** le groupe F est soit un ester, un carbonate, un carbamate ou un éther.

3. Dextran selon l'une quelconque des revendications précédentes caractérisé en ce le dextran est un carboxymethyl dextran (DMC) de formule IV 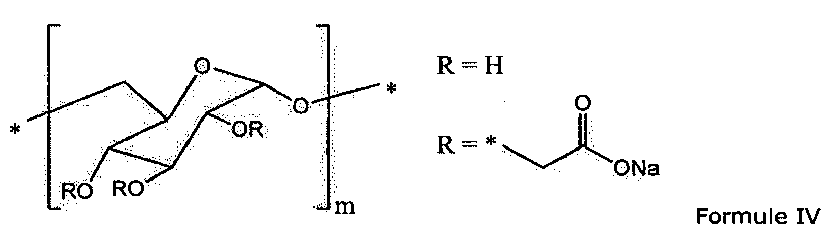 ou l'acide correspondant, et le degré de polymérisation m est compris entre 10 et 10000.

4. Dextran selon l'une quelconque des revendications précédentes caractérisé en ce le dextran est un ester monosuccinique de dextran ou succinicaciddextran (DSA) de formule V 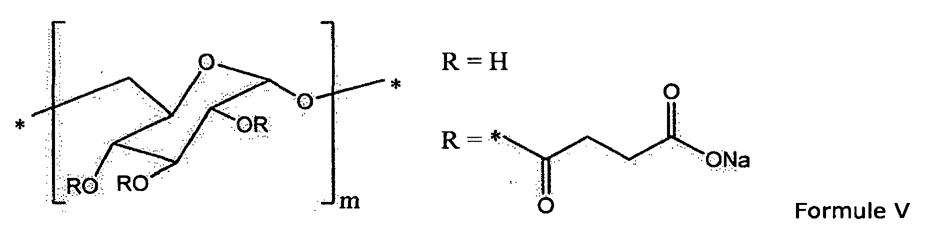 ou l'acide correspondant, et le degré de polymérisation m est compris entre 10 et 10000.

5. Dextran selon l'une quelconque des revendications précédentes **caractérisé en ce que** le groupe R est choisi dans les groupes suivants : 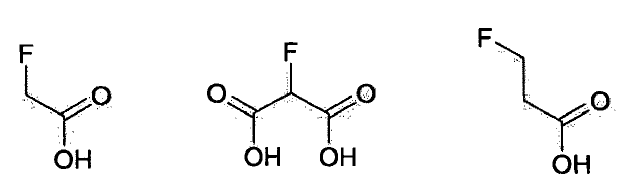 ou leurs sels de cations alcalins.

6. Dextran selon l'une quelconque des revendications précédentes **caractérisé en ce que** l'amino-acide hydrophobe est choisi parmi les dérivés du tryptophane.

7. Dextran selon la revendication 6, **caractérisé en ce que** l'amino-acide hydrophobe est choisi parmi le tryptophane, le tryptophanol, le tryptophanamide, le 2-indole éthylamine et leurs sels de cation alcalin.

8. Dextran selon la revendication 7, **caractérisé en ce que** l'amino-acide hydrophobe est choisi parmi le tryptophane et ses sels de cation alcalin.

9. Dextran selon l'une quelconque des revendications 1 à 5 **caractérisé en ce que** l'amino-acide hydrophobe est choisi parmi la phenylalanine, la leucine, l'isoleucine et la valine et leurs dérivés alcool, amide ou décarboxylés.

10. Dextran selon l'une quelconque des revendications précédentes **caractérisé en ce que** son degré de polymérisation m est compris entre 10 et 1000.

11. Dextran selon l'une quelconque des revendications 1 à 8 **caractérisé en ce que** l'amino-acide hydrophobe est le sel de sodium du tryptophane.

12. Composition pharmaceutique **caractérisée en ce qu'**elle comprend un dextran selon l'une quelconque des revendications précédentes et au moins un principe actif choisi dans le groupe constitué par les protéines, les glycoprotéines, les peptides et les molécules thérapeutiques non-peptidiques.

## Patentansprüche

1. Funktionalisiertes Dextran, **dadurch gekennzeichnet, dass** es die folgende allgemeine Formel aufweist: 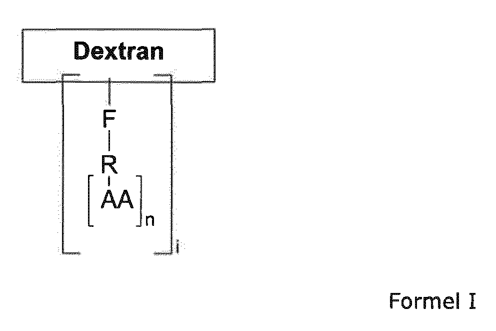 wobei
• R eine Kette umfassend zwischen 1 und 18 Kohlenstoffatomen ist, optional verzweigt und/oder ungesättigt, umfassend ein oder mehrere Heteroatome, und wenigstens eine Säurefunktion aufweist,
• F entweder ein Ester, ein Thioester, ein Amid, ein Carbonat, ein Carbamat, ein Ether, ein Thioether oder ein Amin ist,
• AA ein Rest einer L oder D hydrophoben Aminosäure, ist, hergestellt durch Verknüpfung zwischen dem Amin der Aminosäure und einer Säure, die von der Gruppe R getragen ist, wobei der Aminosäurerest ausgewählt ist aus den Derivaten von Tryptophan, oder aus Phenylalanin, Leucin, Isoleucin und Valin und deren Alkohol-, Amid- oder decarboxylierten Derivaten,
i den Molenbruch des Substituenten F-R-[AA]ₙ pro Glycosid-Einheit darstellt und zwischen 0,1 und 2 beträgt,
n den Molenbruch der R darstellt, die durch AA substituiert sind, und zwischen 0,05 und 1 beträgt,
sofern R nicht durch AA substituiert ist, die Säure(s) der Gruppe R kationische Carboxylate sind, und
das Dextran bei neutralem pH amphiphil ist.

2. Dextran nach Anspruch 1, **dadurch gekennzeichnet, dass** die Gruppe F entweder ein Ester, ein Carbonat, ein Carbamat oder ein Ether ist.

3. Dextran nach einem der vorangehenden Ansprüche, **dadurch gekennzeichnet, dass** das Dextran ein Carboxymethyldextran (DMC) der Formel IV 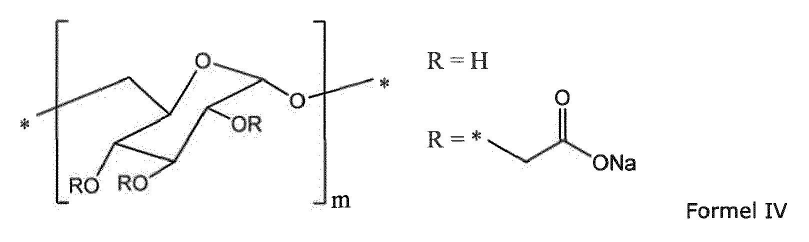 oder der korrespondierenden Säure ist, und der Polymerisationsgrad m zwischen 10 und 10000 beträgt.

4. Dextran nach einem der vorangehenden Ansprüche, **dadurch gekennzeichnet, dass** das Dextran ein Monosuccinatester von Dextran oder Bernsteinsäuredextran (DSA) der Formel V 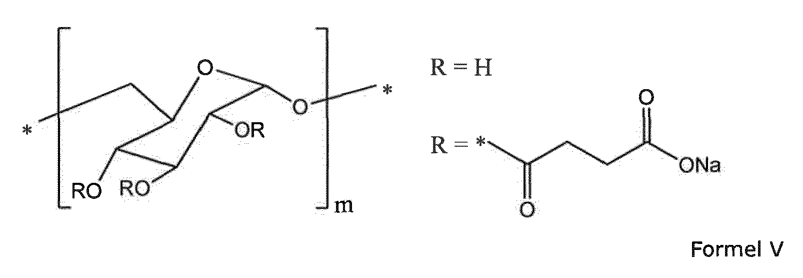 oder der korrespondierenden Säure ist, und der Polymerisationsgrad m zwischen 10 und 10000 beträgt.

5. Dextran nach einem der vorangehenden Ansprüche, **dadurch gekennzeichnet, dass** die Gruppe R ausgewählt ist aus den folgenden Gruppen: 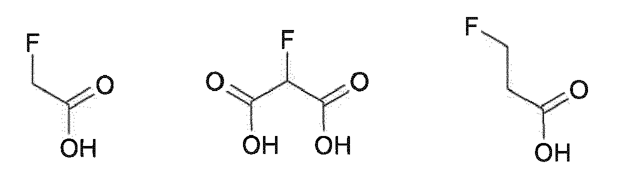 oder deren Salzen aus alkalischen Kationen.

6. Dextran nach einem der vorangehenden Ansprüche, **dadurch gekennzeichnet, dass** die hydrophobe Aminosäure ausgewählt ist aus den Derivaten von Tryptophan.

7. Dextran nach Anspruch 6, **dadurch gekennzeichnet, dass** die hydrophobe Aminosäure ausgewählt ist aus Tryptophan, Tryptophanol, Tryptophanamid, 2-Indolethylamin und deren Salzen aus alkalischen Kationen.

8. Dextran nach Anspruch 7, **dadurch gekennzeichnet, dass** die hydrophobe Aminosäure ausgewählt ist aus Tryptophan und dessen Salzen aus alkalischen Kationen.

9. Dextran nach einem der Ansprüche 1 bis 5, **dadurch gekennzeichnet, dass** die hydrophobe Aminosäure ausgewählt ist aus Phenylalanin, Leucin, Isoleucin und Valine, und deren Alkohol-, Amid- oder decarboxylierten Derivaten.

10. Dextran nach einem der vorangehenden Ansprüche, **dadurch gekennzeichnet, dass** sein Polymerisationsgrad m zwischen 10 und 1000 beträgt.

11. Dextran nach einem der Ansprüche 1 bis 8, **dadurch gekennzeichnet, dass** die hydrophobe Aminosäure das Natriumsalz von Tryptophan ist.

12. Pharmazeutische Zusammensetzung, **dadurch gekennzeichnet, dass** sie ein Dextran nach einem der vorangehenden Ansprüche und wenigstens einen Wirkstoff umfasst, der ausgewählt ist aus der Gruppe bestehend aus Proteinen, Glykoproteinen, Peptiden und nicht-peptidischen therapeutischen Molekülen.

## Claims

1. Functionalized dextran, **characterized in that** it corresponds to the following general formula: 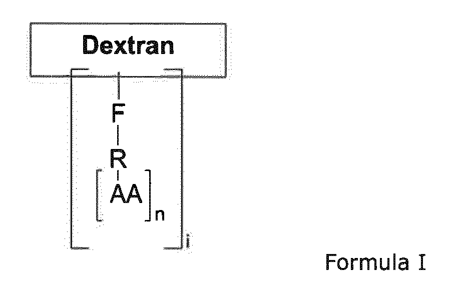
• R being an optionally branched and/or unsaturated chain comprising between 1 and 18 carbon atoms, comprising one or more heteroatoms and having at least one acid function,
• F being either an ester, a thioester, an amide, a carbonate, a carbamate, an ether, a thioether or an amine,
• AA being a hydrophobic L or D amino acid residue which is a product of the coupling between the amine of the amino acid and an acid borne by the R group, said amino acid residue being selected from tryptophan derivatives, or from phenylalanine, leucine, isoleucine and valine and their alcohol, amide or decarboxylated derivatives,
i represents the molar fraction of F-R-[AA]ₙ substituent per glycoside unit and is between 0.1 and 2,
n represents the molar fraction of the R groups which are substituted by AA and is between 0.05 and 1,
when R is not substituted by AA, then the acid(s) of the R group is(are) cationic carboxylate(s),
said dextran being amphiphilic at neutral pH.

2. Dextran according to Claim 1, **characterized in that** the F group is either an ester, a carbonate, a carbamate or an ether.

3. Dextran according to either one of the preceding claims, **characterized in that** the dextran is a carboxymethyl dextran (DMC) of formula IV 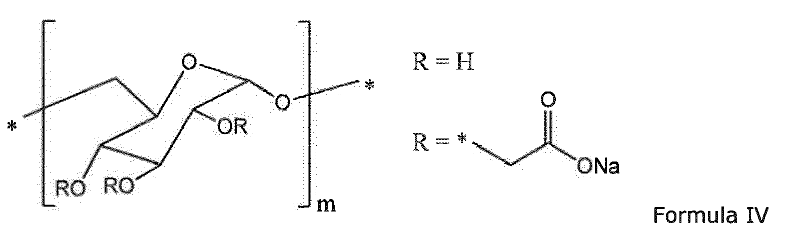 or the corresponding acid, and the degree of polymerization m is between 10 and 10 000.

4. Dextran according to any one of the preceding claims, **characterized in that** the dextran is a dextran monosuccinic ester or dextran succinic acid (DSA) of formula V 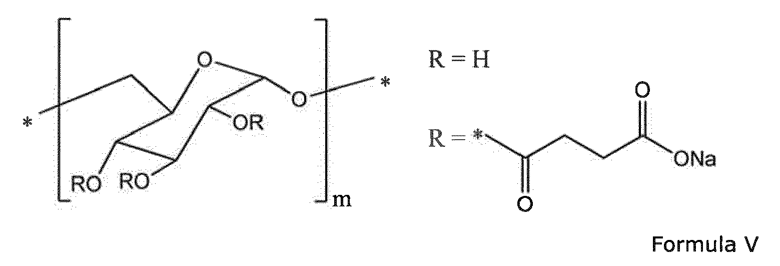 or the corresponding acid, and the degree of polymerization m is between 10 and 10 000.

5. Dextran according to any one of the preceding claims, **characterized in that** the R group is selected from the following groups: 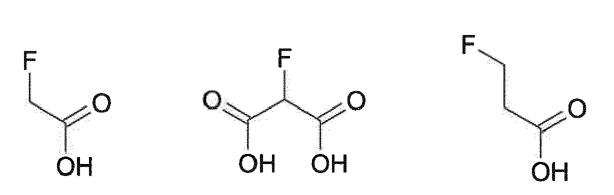 or their salts of alkali metal cations.

6. Dextran according to any one of the preceding claims, **characterized in that** the hydrophobic amino acid is selected from tryptophan derivatives.

7. Dextran according to Claim 6, **characterized in that** the hydrophobic amino acid is selected from tryptophan, tryptophanol, tryptophanamide, 2-indole ethylamine and their salts of alkali metal cations.

8. Dextran according to Claim 7, **characterized in that** the hydrophobic amino acid is selected from tryptophan and its salts of alkali metal cations.

9. Dextran according to any one of Claims 1 to 5, **characterized in that** the hydrophobic amino acid is selected from phenylalanine, leucine, isoleucine and valine and their alcohol, amide or decarboxylated derivatives.

10. Dextran according to any one of the preceding claims, **characterized in that** its degree of polymerization m is between 10 and 1000.

11. Dextran according to any one of Claims 1 to 8, **characterized in that** the hydrophobic amino acid is the sodium salt of tryptophan.

12. Pharmaceutical composition, **characterized in that** it comprises a dextran according to any one of the preceding claims and at least one active ingredient selected from the group consisting of proteins, glycoproteins, peptides and non-peptide therapeutic molecules.
